Europäisches Patentamt

European Patent Office

Office européen des brevets

(11) Veröffentlichungsnummer: **0 224 218**
**B1**

# (12) EUROPÄISCHE PATENTSCHRIFT

(45) Veröffentlichungstag der Patentschrift:
**02.08.89**

(21) Anmeldenummer: **86116186.7**

(22) Anmeldetag: **22.11.86**

(51) Int. Cl.⁴: **C07C 45/71**, C07C 49/04

(54) Verfahren zur Herstellung von Methylisopropylketon und Diethylketon.

(30) Priorität: **29.11.85 DE 3542297**

(43) Veröffentlichungstag der Anmeldung:
**03.06.87 Patentblatt 87/23**

(45) Bekanntmachung des Hinweises auf die Patenterteilung:
**02.08.89 Patentblatt 89/31**

(84) Benannte Vertragsstaaten:
**BE CH DE FR GB IT LI NL**

(56) Entgegenhaltungen:
**EP-A- 0 166 490**
**DE-A- 2 257 675**

(73) Patentinhaber: **BASF Aktiengesellschaft,
Carl-Bosch-Strasse 38, D-6700 Ludwigshafen(DE)**

(72) Erfinder: **Luksza, Michael, Dr., Gaustrasse 54 a,
D-6702 Bad Dürkheim(DE)**
Erfinder: **Lenz, Hans-Heinrich, Dr., Geminiweg 2,
D-4950 Minden(DE)**

**Beschreibung**

Die vorliegende Erfindung betrifft ein Verfahren zur Herstellung von Methylisopropylketon und Diethylketon durch Umsetzung von Ethylmethylketon mit Methanol in der Gasphase in Gegenwart eines Katalysators.

Es ist bekannt, daß höhere Ketone aus den jeweils niedrigeren Homologen aufgebaut werden können. Im Falle der Methylierung kann man die als Edukt verwendeten Ketone in einer Aldolreaktion mit Formaldehyd umsetzen, aus den entstehenden Additionsprodukten Wasser abspalten und die C=C-Doppelbindung der resultierenden α-β-ungesättigten Ketone abschließend einer Hydrierung unterwerfen.

Eine andere Möglichkeit besteht darin, die jeweils unumgesetzten Ketone in ihre Enolate zu überführen und diese mit Alkylhalogeniden - bevorzugt den Bromiden oder Iodiden - zu alkylieren. Dazu müssen jedoch meist stöchiometrische Mengen alkalischer Substanzen, wie Alkalimetall, -alkoholat, -hydroxid oder -amid, verwendet werden, vgl. Houben-Weyl, "Methoden der organischen Chemie", 4. Auflage, Band 7/26, S. 1385ff.

Der offensichtliche Nachteil dieser Alkylierungsverfahren liegt in der Verwendung von teilweise kostspieligen Hilfsbasen und damit in der Bildung von stöchiometrischen Mengen Alkali- oder Ammoniumhalogeniden, die nur umständlich in Halogenanteil und freie Base rückgespalten werden können. Damit ergibt sich neben der problematischen Weiterverwendung der anfallenden Nebenprodukte eine wenig umweltfreundliche Aufarbeitung. Weiterhin handelt es sich bei diesen Verfahren größtenteils um mehrstufige Prozesse, bei denen die Wertprodukte oft nur in schlechten Ausbeuten und geringer Selektivität entstehen.

Aus DE-A-22 57 675 ist die Umsetzung von Aceton mit Methanol in der Gasphase an einem Trägerkatalysator bekannt, die zu einem Gemisch höherer Ketone führt.

Nachteilig ist hier der Einsatz eines aufwendig herzustellenden und aufgrund der Verwendung von Metallsalzen wie Cu(NO₃)₂ und AgNO₃, teueren Katalysatorsystems sowie das Entstehen eines komplexen Ketongemisches bei vergleichsweise geringeren Einzelselektivitäten. So entsteht bei der Umsetzung von Methylethylketon mit Methanol (vgl. DE-A-22 57 675, Bsp. 13) bei einem 77 Molprozentigen Umsatz an Methylethylketon Methylisopropylketon in 22,1 Mol-prozentiger und Diethylketon in 14,6 Mol-prozentiger Ausbeute.

Der Erfindung liegt die Aufgabe zugrunde, ein Verfahren der eingangs genannten Art zur Verfügung zu stellen, bei dem Methylisopropylketon und/oder Diethylketon in hohen Ausbeuten und guten Selektivitäten erhalten werden.

Diese Aufgabe wird gelöst, durch ein Verfahren der genannten Art, das dadurch gekennzeichnet ist, daß man bei einer Temperatur von 350 bis 500°C arbeitet und als Katalysator ein Metalloxid, ausgewählt unter den Oxiden von Cer, Chrom, Eisen, Magnesium und/oder Mangan einsetzt, wobei man ein Gemisch von Methylisopropylketon und Diethylketon erhält, und dieses gegebenenfalls in die Einzelverbindungen auftrennt.

Nach einer bevorzugten Ausführungsform setzt man als Katalysator Magnesiumoxid ein. Besonders zweckmäßig wird bei Temperaturen zwischen 450 und 500°C und einem Druck zwischen 1 und 20 bar gearbeitet. Nach einer weiteren bevorzugten Ausführungsform führt man die Umsetzung mit einem Molverhältnis Keton : Methanol von 1 : 1 bis 1 : 3 durch. Es ist ferner bevorzugt, in Gegenwart von Wasser zu arbeiten.

Es wurde erfindungsgemäß überraschenderweise gefunden, daß durch die Umsetzung von Methylethylketon mit Methanol und Wasser in der Gasphase bei einer Temperatur von 350 bis 500°C und einem Druck von 1 bis 20 bar in Gegenwart eines Metalloxids, ausgewählt aus der Gruppe bestehend aus den Oxiden der Metalle Cer, Chrom, Eisen, Magnesium und/oder Mangan, Methylisopropylketon und Diethylketon, in hohen Ausbeuten und guten Selektivitäten erhalten werden.

Das Verhältnis der beiden Produkte ist durch Änderung der Temperatur und Verweilzeit variierbar. Eine gesteigerte Selektivität an Methylisopropylketon und Diethylketon läßt sich bei einem geringen Umsatz erzielen. Durch Zurückführen des unumgesetzten Methylethylketons kann der Gesamtumsatz jedoch vervollständigt werden. Das ist von Bedeutung bei einer kontinuierlichen Durchführung des erfindungsgemäßen Verfahrens.

Methylethylketon wird vorzugsweise mit einer äquimolaren Menge an Methanol in Gegenwart einer ebenfalls äquimolaren Menge Wasser bei 350 bis 500°C, insbesondere bei 450 bis 480°C, bevorzugt an einem Magnesiumoxidkatalysator umgesetzt.

Das erfindungsgemäße Verfahren wird in Gegenwart eines Katalysators, nämlich eines Metalloxides, ausgewählt aus der Gruppe bestehend aus den Oxiden der Metalle Cer, Chrom, Eisen, Magnesium und Mangan durchgeführt.

Beispielsweise sind zu nennen Cer(IV)oxid, Chrom(III)oxid, Eisen(III)oxid, Magnesiumoxid oder Mangan(II)oxid. Die Verwendung von Magnesiumoxid ist bevorzugt.

Die Herstellung dieser Metalloxide ist bekannt, vgl. US-A-3 972 828 und DE-C-20 24 282. Es handelt sich um handelsübliche Produkte. Wie in der Katalysatortechnik üblich, werden sie vorteilhaft in geformtem Zustand, beispielsweise in Form von Tabletten, Strängen, Pillen, Kugeln oder Ringen eingesetzt.

Die Durchführung des erfindungsgemäßen Verfahrens erfolgt in der Gasphase bei einer Temperatur von 350 bis 500°C, vorzugsweise 450 bis 480°C und bei einem Druck von 1 bis 20 bar, vorzugsweise bei atmosphärischem Druck.

Das Molverhältnis der beiden Reaktanden Keton und Methanol beträgt 1 : 1 bis 1 : 3. Vorzugsweise arbeitet man mit einem Molverhältnis Keton : Methanol von 1 : 1. Es empfiehlt sich ferner, Wasser in einem Molverhältnis Wasser : Keton von 1 : 1 bis 5 : 1 zuzusetzen.

Das erfindungsgemäße Verfahren wird zweckmäßig so durchgeführt, daß man ein Gemisch der Reaktionspartner im genannten Molverhältnis, gegebenenfalls in Anwesenheit von Wasser, zunächst kontinuierlich einem Verdampfersystem zuführt, das eine vollständige Verdampfung der Reaktanden gewährleistet. Die resultierende Gasmischung wird dabei auf eine, mit der im Reaktor benötigten Reaktionstemperatur vergleichbaren Temperatur gebracht. Um eventuelle Wärmeverluste auf dem Weg zum Reaktor auszugleichen, wählt man vorzugsweise eine etwas höhere Temperatur im Verdampfer.

Das Gasgemisch tritt dann in den auf die erforderliche Reaktionstemperatur erhitzten Reaktor ein, wobei es sich als vorteilhaft erweist, das Temperaturprofil über die gesamte Reaktorlänge möglichst gleichmäßig zu halten. Als Reaktor dient dabei vorteilhaft ein Rohrreaktor, in dessen Innenraum sich der Katalysator befindet.

Als typische, jedoch nicht limitierende Katalysatorbelastung wählt man z.B. 250 bis 450 ml (bezogen auf den flüssigen Aggregatzustand) flüssiges Reaktionsgemisch je Liter Katalysator und Stunde.

Die Verweilzeit im Reaktor beträgt vorteilhaft ca. 6 bis 15 sec.

Das den Reaktor verlassende gasförmige Reaktionsgemisch wird anschließend kondensiert und kann durch fraktionierende Destillation gereinigt werden.

Besondere Vorteile des erfindungsgemäßen Verfahrens liegen im gezielten Ablauf der Reaktion, d.h. Nebenprodukte, wie beispielsweise toxische Vinylketone und deren Polymere oder die durch Wasserstofftransfer von Methanol zum Keton entstehenden Alkohole bilden sich nur in geringem Maße.

Weiterhin wird mit einem geringeren Überschuß an Methanol gearbeitet als er im Stande der Technik üblich ist.

Schließlich ist der Umsatz an eingesetztem Keton sehr hoch (65 bis 95 %). Gleichzeitig entstehen die gewünschten Wertprodukte in höherer Gesamtselektivität als im bekannten Verfahren, bei gleichzeitig deutlich höherem Substratdurchsatz.

Die durch das erfindungsgemäße Verfahren erhaltenen methylierten Ketone sind wertvolle Zwischenprodukte für die Synthese von Vitamin E bzw. gesuchte Solventien. Methylisopropylketon ist ferner ein Pflanzenschutz-Vorprodukt.

Die nachfolgenden Beispiele sollen die Erfindung näher erläutern.

Beispiel 1

120 ml eines Gemisches aus 72 g Methylethylketon, 32 g Methanol und 18 g Wasser wurden pro Stunde kontinuierlich bei 550°C verdampft und über 350 ml Magnesiumoxid in Tablettenform geleitet. Die Reaktortemperatur wurde mittels Temperaturregelung über den gesamten Reaktor auf 500°C eingestellt. Das erhaltene gasförmige Reaktionsprodukt wurde kondensiert und anschließend gaschromatographisch analysiert. Nach Abzug von Methanol und Wasser wurde folgendes Ergebnis erhalten (Angabe in Gew.-%): Bei einem 52 Mol-prozentigen Umsatz an Methylethylketon entstanden

21,3 Mol-% Methylisopropylketon und 19,2 Mol-% Diethylketon, entsprechend einer Selektivität von 41,3 beziehungsweise 37,1 %.

Beispiel 2

Unter denselben Bedingungen wie in Beispiel 1 wurden 72 g Methylethylketon, 64 g Methanol und 18 g Wasser über den Reaktor geleitet. Nach Abzug von Methanol wurde folgendes Ergebnis erhalten (Angabe in Gew.-%): Bei einem 78 Mol-prozentigen Umsatz an Methylethylketon entstanden 22,6 Molprozent Methylisopropylketon und 22,6 Molprozent Diethylketon, entsprechend einer Selektivität von jeweils 29,3 %.

**Patentansprüche**

1. Verfahren zur Herstellung von Methylisopropylketon und/oder Diethylketon, durch Umsetzung von Methylethylketon mit Methanol in der Gasphase bei erhöhter Temperatur in Gegenwart eines Katalysators, dadurch gekennzeichnet, daß man bei einer Temperatur von 350 bis 500°C arbeitet und als Katalysator ein Metalloxid, ausgewählt unter den Oxiden von Cer, Chrom, Eisen, Magnesium und/oder Mangan einsetzt, wobei man ein Gemisch von Methylisopropylketon und Diethylketon erhält, und dieses gegebenenfalls in die Einzelverbindungen auftrennt.

2. Verfahren nach Anspruch 1, dadurch gekennzeichnet, daß man als Katalysator Magnesiumoxid einsetzt.

3. Verfahren nach einem der Ansprüche 1 oder 2, dadurch gekennzeichnet, daß man bei Temperaturen zwischen 450 und 500°C arbeitet.

4. Verfahren nach einem der Ansprüche 1 bis 3, dadurch gekennzeichnet, daß man bei einem Druck zwischen 1 und 20 bar arbeitet.

5. Verfahren nach einem der Ansprüche 1 bis 4, dadurch gekennzeichnet, daß man die Umsetzung mit einem Molverhältnis Keton : Methanol von 1 : 1 bis 1 : 3 durchführt.

6. Verfahren nach einem der Ansprüche 1 bis 5, dadurch gekennzeichnet, daß man in Gegenwart von Wasser arbeitet.

**Claims**

1. A process for the preparation of methyl isopropyl ketone and/or diethyl ketone by reacting methyl ethyl ketone with methanol in the gas phase at elevated temperatures in the presence of a catalyst, wherein the reaction is carried out at from 350 to 500°C and the catalyst used is a metal oxide selected from the oxides of cerium, chromium, iron, magnesium and manganese, a mixture of methyl isopropyl ketone and diethyl ketone being obtained and, if required, being separated into the individual compounds.

2. A process as claimed in claim 1, wherein the catalyst used is magnesium oxide.

3. A process as claimed in either of claims 1 and 2, which is carried out at from 450 to 500°C.

4. A process as claimed in any of claims 1 to 3, which is carried out at from 1 to 20 bar.

5. A process as claimed in any of claims 1 to 4, wherein the reaction is carried out using a molar ratio of ketone to methanol of from 1:1 to 1:3.

6. A process as claimed in any of claims 1 to 5, which is carried out in the presence of water.

**Revendications**

1. Procédé de préparation de méthylisopropylcétone et/ou de diéthylcétone, par réaction de méthyléthylcétone avec le méthanol en phase gazeuse à températures élevées en présence d'un catalyseur, caractérisé en ce qu'on travaille à une température de 350 à 500°C et on utilise comme catalyseur un oxyde métallique choisi par les oxydes de cérium, de chrome, de fer, de magnésium et/ou de manganèse, en obtenant un mélange de méthylisopropylcétone et de diéthylcétone, et on sépare éventuellement celui-ci en ses composants individuels.

2. Procédé selon la revendication 1, caractérisé en ce qu'on utilise en tant que catalyseur de l'oxyde de magnésium.

3. Procédé selon l'une ou l'autre des revendications 1 et 2, caractérisé en ce qu'on travaille à des températures comprises entre 450 et 500°C .

4. Procédé selon l'une quelconque des revendications 1 à 3, caractérisé en ce qu'on travaille à des pressions comprises entre 1 et 20 bar.

5. Procédé selon l'une quelconque des revendications 1 à 4, caractérisé en ce qu'on effectue la réaction avec un rapport molaire de la cétone au méthanol de 1:1 à 1:3.

6. Procédé selon l'une quelconque des revendications 1 à 5, caractérisé en, ce qu'on travaille en présence d'eau.